# EUROPEAN PATENT APPLICATION

(11) **EP 3 023 053 A1**
(43) Date of publication of application: **25.05.2016**
(21) Application number: 14194258.1
(22) Date of filing: 21.11.2014
(51) Int. Cl.: A61B 5/024, A61B 5/00, A61B 5/1455

(54) **Apparatus and method for sensing light**

(71) Applicant: Nokia Technologies OY, 02610 Espoo (FI)
(72) Inventor: Lasarov, Harri, 02280 Espoo (FI); Salo, Antti, 08500 Lohja as. (FI)
(74) Representative: Nokia Corporation

(57) **Abstract**

Examples of the present disclosure relate to an apparatus and method for sensing light. The apparatus comprises a support structure configured to attach to an appendage of a user, wherein the support structure comprises: a light source; an optical sensing element; and a covering element arranged to cover at least the optical sensing element, wherein the covering element is configured such that the covering element's transmissivity is adjustable.

## Description

### TECHNOLOGICAL FIELD

Examples of the present disclosure relate to an apparatus and method for sensing light. Some examples, though without prejudice to the foregoing, relate to a wearable apparatus for sensing light. In yet further examples, a wrist wearable apparatus may be provided that is configured for use in determining biometric/physiological measurements, not least for example heart rate, via photoplethysmography.

### BACKGROUND

Optical heart rate monitors may use photoplethysmography (PPG) to measure a user's pulse. A PPG sensor may comprise an LED and a photodetector configured such that light from the LED is directed towards a user's skin and light reflected from the user's tissue is detected by the photodetector. Changes in the tissue, such as pulsating blood, can be determined based on a variation in the amount of the reflected light detected.

Conventional wearable heart rate monitoring devices and PPG sensors for the same may not always be optimal. Typically, in various prior devices, a protective window is provided that covers the LED and photodetector. The window is transparent so as to allow light to be emitted and detected therethrough. However, this also means that various internal components, not least for example the LED and photodetector, are visible, particularly when the device is not in use/not being worn by a user. This can be aesthetically un-appealing and undesirable from a product design point of view. This can also affect a product designer's design freedom when seeking to design an efficient and yet aesthetically appealing wearable heart rate monitoring device.

The listing or discussion of any prior-published document or any background in this specification should not necessarily be taken as an acknowledgement that the document or background is part of the state of the art or is common general knowledge. One or more aspects/examples of the present disclosure may or may not address one or more of the background issues.

### BRIEF SUMMARY

According to at least some but not necessarily all examples of the disclosure there is provided an apparatus comprising: a support structure configured to attach to an appendage of a user, wherein the support structure comprises: a light source; an optical sensing element; and a covering element arranged to cover at least the optical sensing element, wherein the covering element is configured such that the covering element's transmissivity is adjustable.

According to at least some but not necessarily all examples of the disclosure there is provided a method comprising: adjusting a transmissivity of a covering element of an apparatus, wherein the apparatus comprises: a support structure configured to attach to an appendage of a user, wherein the support structure comprises: a light source; an optical sensing element; and wherein the covering element is arranged to cover at least the optical sensing element.

According to at least some but not necessarily all examples of the disclosure there is provided an apparatus comprising means configured to cause/enable the apparatus at least to perform the above method.

According to at least some but not necessarily all examples of the disclosure there is provided an apparatus comprising: means configured to attach the apparatus to an appendage of a user; means configured to emit light; means configured to detect light; means configured to cover at least the light detection means, wherein the covering means is configured such that its transmissivity is adjustable.

### BRIEF DESCRIPTION OF THE DRAWINGS

For a better understanding of various examples of the present disclosure that are useful for understanding the detailed description and certain embodiments of the invention, reference will now be made by way of example only to the accompanying drawings in which:
Figure 1 schematically illustrates an example of an apparatus according to the present disclosure;
Figures 2A and 2B schematically illustrate first and second transmissivity states of an example of a covering element according the present disclosure;
Figures 3A and 3B schematically illustrate a further example of an apparatus according to the present disclosure;
Figures 4A and 4B schematically illustrate yet a further example of an apparatus according to the present disclosure;
Figure 5 schematically illustrates a yet further example of an apparatus according to the present disclosure; and
Figure 6 schematically illustrates an example of a method according to the present disclosure.

### DETAILED DESCRIPTION

The Figures schematically illustrate an apparatus 100 comprising:
a support structure 101 configured to be attachable to an appendage of a user, wherein the support structure comprises:
a light source 102;
an optical sensing element 103; and
a covering element 104 arranged to cover at least the optical sensing element, wherein the covering element is configured such that the covering element's transmissivity is adjustable.

In some non-limiting examples, the covering element's transmissivity may be adjusted so as to be switchable between a substantially transparent transmissivity state and a substantially opaque transmissivity state. For example, when the apparatus is attached to a user or in use (e.g. a measurement from the optical sensing element is to be made), the covering element's transmissivity may be adjusted such that it is substantially transparent so as to allow light to pass through the covering element for detection by the optical sensing element. Whereas, when the apparatus is not attached to the user or not in use, the covering element's transmissivity may be adjusted such that it is substantially opaque/ non transparent and blocks the transmission of light therethrough. This may enable at least the optical sensing element to be non-visible/concealed/hidden from a user's view, or at least reduce the visibility of the same, when the apparatus is not attached to the user or not in use.

In some non-limiting examples, the covering element may be selectively opaque during non-use to conceal internal components of the apparatus and selectively transparent during use to enhance light transmissivity during use. This may improve the detection efficiency as well as reduce light source brightness requirements and energy requirements (and thus enhancing operating time / battery lifetime) that might otherwise be required if instead, for example, the covering element were to be permanently tinted to conceal/reduce visibility of internal components.

Example of apparatuses according to the present disclosure will now be described with reference to the Figures. Similar reference numerals are used in the Figures to designate similar features. For clarity, all reference numerals are not necessarily displayed in all figures.

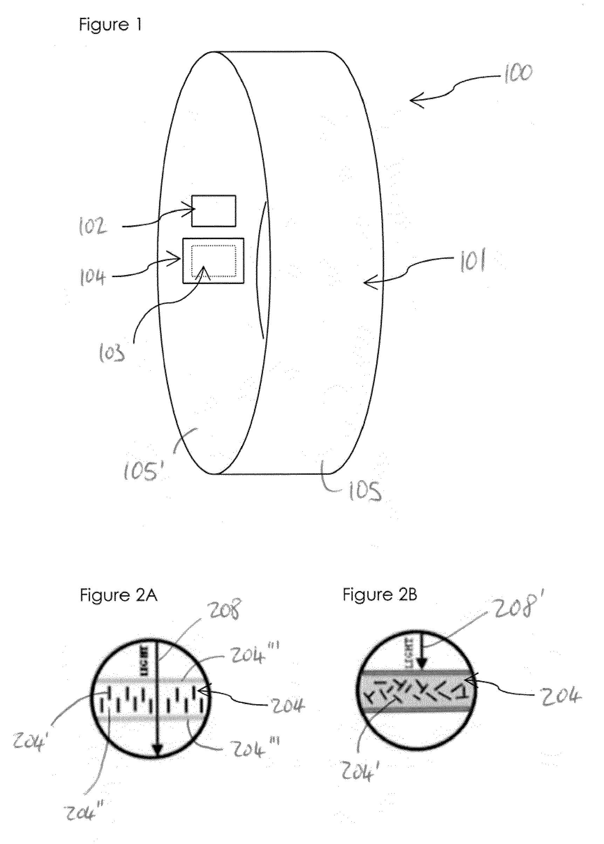

Figure 1 schematically illustrates a block diagram of an apparatus 100 according to an example of the present disclosure. Figure 1 focuses on the functional components necessary for describing the operation of the apparatus 100.

The apparatus 100 comprises a support structure 101 that is configured to attach to an appendage of a user such that the apparatus is configured for wearable use. In some examples, the support structure 101 may correspond to a means configured to attach the apparatus to an appendage of a user. The support structure may be configured to attach the user's appendage via any suitable means and attachment mechanism. In some examples, the support structure may be configured to least partially enclose an appendage of a user, or it may be configured to attach to user's appendage via other attachment means not least for example via an attachment mechanism or adhesion (e.g. a bandage, plaster, patch adhered to skin). In some examples, the support structure may take the form of a wearable band, wrist strap or bracelet like structure that can be worn on a user's appendage. In some examples the user's appendage may correspond to a user's limb or wrist. In some examples the support structure may comprise at least a part of an external housing for the apparatus and/or a structural framework onto which various internal components, electronics and elements of the apparatus may be attached and mounted to. Such internal components and elements of the apparatus may correspond not least for example to: the optical sensing element, the light source as well as other components such as circuitry, (flexible) printed circuit boards, internal power supply ...

The support structure comprises a light source 102. In some examples, the light source may correspond to a means configured to emit light at least in the visible part of the electromagnetic spectrum and/or IR light. In some examples the light source may comprise one or more Light Emitting Diodes (LED's). The light source may generate and/or give rise to the emission of light of particular frequency ranges, not least for example light of particular colours or specific parts of the electromagnetic spectrum (for example via the use of: red, green, blue and infrared LED's or via the use of red, green, blue and infrared filters).

The support structure also comprises an optical sensing element 104. In some examples, the optical sensing element may correspond to a means configured to detect light, not least for example, a photo detector, photodiode, photo sensor or light sensor.

In some examples, the optical sensing element and light source are configured so as to provide a PPG sensor or a PPG sensor module.

The support structure also comprises a covering element 104. The covering element is arranged so as to cover at least the optical sensing element. In some examples, the covering element may correspond to a means configured to cover a least the optical sensing element. In some examples, the covering element may be configured to provide a protective cover or window over at least the optical sensing element, or a coating for such a protective layer/window.

In some examples, the covering element may be configured to cover additionally the light source and/or additional light sources, optical sensing elements or other elements of the apparatus. In some examples a single covering element may be provided to cover a plurality of elements and components. In some examples, a separate covering element is provided for covering a plurality of elements and components such that a plurality of covering elements may be provided to cover a plurality of elements and components.

In some examples, the covering element may comprise part of an external housing for the apparatus. In some examples, an outer surface of the covering element may form an external surface of the apparatus. In some examples, the covering element may form a window or a covering layer for an aperture in an external housing of the apparatus and may provide a protective barrier to the optical sensing element and the light source. The covering element may be attached to the support structure and a housing of the apparatus in such a manner so as to seal the optical sensing element and light source within the apparatus thereby protecting such components against dust and moisture.

The covering element 104 is configured such that its transmissivity is adjustable. In some examples, the covering element may comprise one or more of the following:
smart glass or switchable glass which may be passively or actively switched between different transmissivity states;
a photochromatic material, whose transmissivity is responsive to incident electromagnetic radiation, not least for example ultraviolet rays (thus it can be 'passively' switched when exposed to ambient UV rays, i.e. when not worn by a user);
a thermochromic material, whose transmissivity is responsive temperature/heat. The thermochromic material may be configured such that its activation temperature for switching between substantially transparent and substantially opaque states may be set at a temperature between ambient temperature and skin temperature (thus it can be 'passively' switched when the apparatus is worn by a user). In other examples, the light source of the apparatus may additionally generate heat when emitting light. The apparatus may be configured such that the thermochromic material is activated by excess heat generated by the light source when it is on. This may enable the use of otherwise wasted energy/heat to switch the thermochromic material to a substantially transparent state when the apparatus/light source is in use;
an electrochromic material whose transmissivity is responsive to an applied voltage/electric field (thus can be 'actively' switched);
a liquid crystal or polymer dispersed liquid device whose transmissivity is responsive to an applied voltage/electric field (thus can be 'actively' switched);; and
a suspended particle device (SPD) whose transmissivity is responsive to an applied voltage/electric field (thus can be 'actively' switched).

Various of the above materials may be provided as ink or pigment to the covering element medium. The covering element may also be provided with an anti-reflective coating on one or more of its surfaces which may further increase its optical transparency.

In some examples, light focussing means such as a lens or other means for redirecting the path of light, may be provided so to focus light from the light source to a part of the user's body and/or focus light reflected from a part of the user's body to the optical sensing element.

The apparatus, when in use and worn by a user, may be configured such that the light source emits light which is directed towards a part of the user appendage, e.g. tissue beneath skin on a user's wrist. The optical sensing element may be configured so as to detect light which is reflected from the part of the appendage. An analysis of the detected reflected light luminosity and intensity as well as variations in the same may be used to determine one or more of: a biometric measurement, a physiological measurement, a photoplethysmogram measurement, volumetric measurement, a blood oxygen saturation measurement, a cardiovascular measurement, a heart rate measurement or a pulse oxygen measurement.

In the apparatus of Figure 1, the apparatus is configured as a wrist wearable device. The support structure takes the form of a band/bracelet device which encloses/wraps around a user's wrist. The support structure defines an outer side 105 and an inner side 105 which is disposed towards the user's wrist and, when worn by a user, is adjacent to or abuts the user's wrist. The light source and optical sensing element are disposed on the inner side of the apparatus such that the light source can emit light toward the user's wrist and such that the optical sensing element can detect the light that is emitted from the light source and reflected by tissue within the user's wrist.

Figures 2A and 2B show a close up detail of a covering element 204 in each of a first and second transmissivity state.

The covering element 204 comprises a material or medium which is configured so as to have adjustable transmissivity. The covering element may be switchable between at least the first transmissivity state and a second transmissivity state. The first transmissivity state may have a first degree of transmissivity which is different to a second degree of transmissivity for the second state. For example, the first state's transmissivity, at least for visible and/or IR wavelengths emitted by the light source, is more transmissive/translucent/transparent than the second state.

In some examples, the second state's transmissivity may be such that it has a low transmissivity to visible wavelengths (not least for example light of one or more of: red, green and blue wavelengths) but a high transmissivity to non-visible wavelengths (not least for example light of IR wavelengths). In such a second state, the covering element may appear to be non-transparent to the human eye, i.e. at visible wavelengths, thereby concealing from a user's view elements lying beneath the covering element, but the covering element may be transparent to non-visible wavelengths such as IR wavelengths.

In the first state shown in Figure 2A, the covering element 204 is configured such that enough light can pass through covering element to enable the sensing of the light. In some examples the first state may be substantially transparent/translucent so that incident light 208 is able to pass through the covering element/material. In the second transmissivity state shown in Figure 2B the covering element's material has been switched to a second different transmissivity state configured to obstruct the path of light so as to reduce the visibility of parts of the apparatus covered by the covering element. In some examples the second state may be substantially opaque/non translucent such that the covering element provides an optically blocking barrier which prevents light 208' passing/being transmitted therethrough.

In the covering element 204 in Figures 2A and 2B, the covering element comprises a suspended particle device (SPD). A thin laminate of rod like nano-scale particles 204' is suspended in a liquid medium 204" and placed between two substrates 204"', such as glass or plastic. In some examples, the liquid comprising the suspended particles may be applied to just a single layer of a substrate.

When a voltage or electric field is applied to the SPD 204, the suspended particles 204' align and enable incident light 208 to pass through the SPD 204. Varying the voltage/electric field applied varies the orientation and degree of alignment of the suspended particles 204' thereby regulating the tint of the SPD and the amount of light transmitted there through. When no voltage is applied, the suspended particles 204' are randomly organised as shown in Figure 2B, thereby blocking and absorbing incident light 208'.

In some examples, the covering element may be configured such that it is in a first state when the apparatus is in use and in a second state when the apparatus is not in use. The apparatus being in use may correspond to at least one or more of:
the apparatus being attached to/worn by a user;
the optical sensing element and/or light source being activated/operational to detect and emit light respectively.

The first state may correspond to a substantially transparent state or at least to a state whereby enough light is able to pass through the covering element for sensing light intensity. Since the covering element is disposed so as to cover at least the optical sensing element, in the first state, light may be permitted to pass through the covering element for detection by the optical sensing element. Where the covering element is configured also to cover the light source, in the first state, light emitted from the light source may pass through the covering element, such that it may be directed to and reflected from a part of a user's appendage for detection by the optical sensor.

In the second state, the covering element may be substantially opaque or at least a state where incident light may be blocked/scattered by the covering element such that light is not able to pass through the covering element. Where the covering element covers and provides a protective window for the optical sensing element, in the second state, the optical sensing element may not be visible through the covering element, or at least its visibility by a user may be reduced as compared to its visibility when the covering element is in the first state. Where the covering element covers the light source, in the second state the light source may also be obscured/not visible through the covering element.

In the second state, elements and internal components covered by the covering element may be non-visible through the covering element and thus obscured and hidden from view of a user thereby hiding such elements beneath the covering element.

The second transmissivity/blocking state may be configured so as to be black or another colour. The apparatus may be configured such that the covering element's appearance and colour in the second state is substantially similar to a colour and/or appearance of adjacent/surrounding elements of the apparatus, such as an adjacent external surface of a housing of the apparatus in which the covering element is disposed.

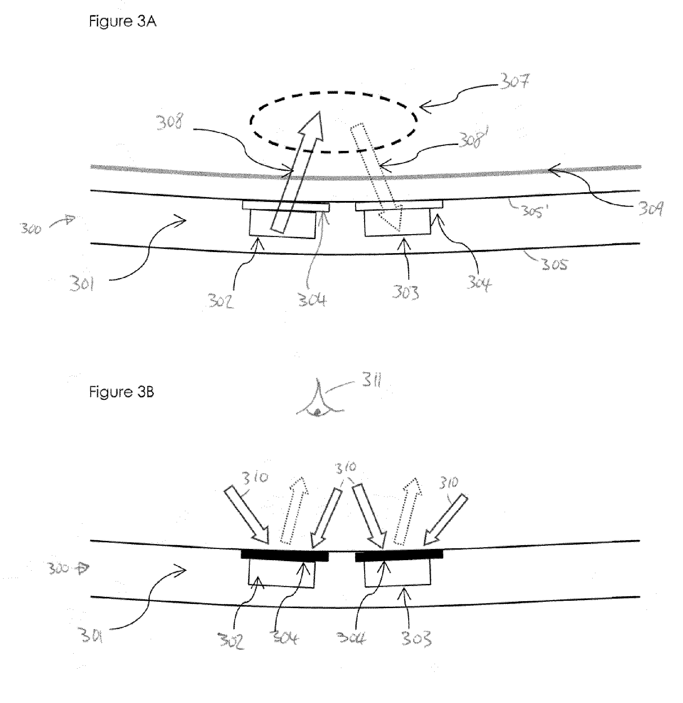

Figure 3A shows a close up partial cross sectional view of a further example of an apparatus 300 according to the present disclosure. The apparatus is shown in use and worn by a user around an appendage of the user 309, such as a user's wrist. The apparatus 300 comprises a support structure 301, a light source 302 and an optical sensing element 303 similar to those described above. Two covering elements 304 are provided, one covering the light source and another covering the optical sensing element. In other examples, a single covering element may be provided which covers both the light source and the optical sensing element.

The covering elements 304 are configured in a first transmissivity state wherein they are substantially transparent/translucent so as to enable enough light to pass through for sensing light intensity. For instance, light 308 emitted from light source 302 can pass through the first covering element 304. The apparatus may be configured such that the light 308 is directed towards a particular part of a user's wrist, i.e. a region 307 where optimal PPG measurements might be made. The light 308 may be reflected from the region 307. Such reflected light 308' may be detected by the optical sensing element 303 having passed through the second covering element 304 which is in the first/transmissive state.

Figure 3B shows the apparatus 300 when not in use. Here the apparatus is no longer being worn by a user. The light source and optical sensing element are inactivated/non-operational/powered down so as to not emit light nor detect light. In such a non-use state of the apparatus, the covering elements 304 may be configured so as to be in the second transmissivity state. This state may comprise a low transmissivity state wherein parts of the apparatus underneath the covering element, such as the light source and optical sensor, are not visible to an external viewer/user, as schematically represented by 311. In the second transmissivity state incident ambient light 310 may be blocked, scattered, reflected or otherwise prevented from passing/transmitting through the covering elements thereby reducing the visibility of the parts of the apparatus lying underneath the covering element making them less clearly visibly to a user than in the first transmissivity state.

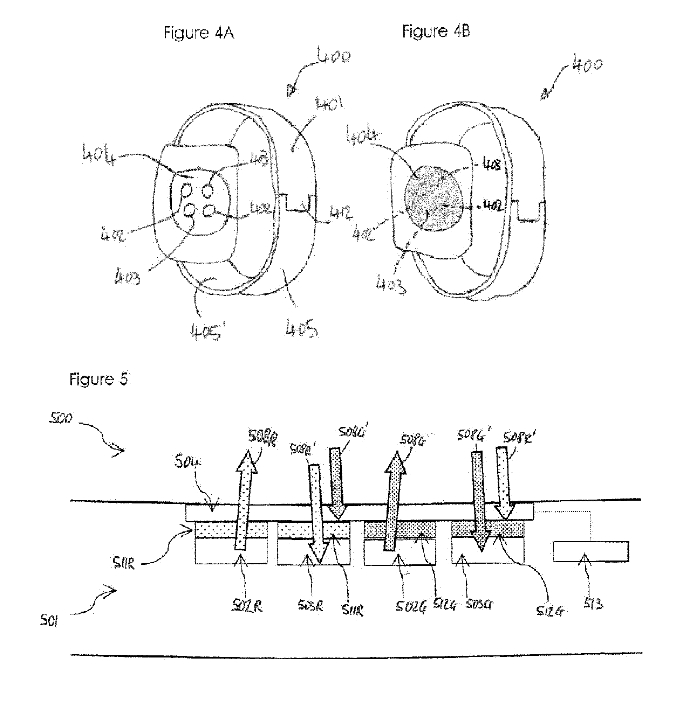

Figures 4A and 4B show a further example of an apparatus 400. The apparatus is provided in the form of a wrist wearable device. The covering element 404 extends over a plurality of light sources 402 and a plurality of light sensing light elements 403, i.e. the covering element not only covers the light sources and light sensing elements but also covers an area outside/surrounding the light sources and light sensing elements. The covering element 404 forms part of an external part of a housing of the apparatus and is provided on an inner side 405' of the apparatus which is adjacent/proximal to the user's wrist when worn.

Figure 4A shows the apparatus 400 in a first/transmissive/in use state wherein the covering element 404 is substantially transparent. In this state, light emitted from the light sources may pass through the covering element so as to be incident on a part of the user's appendage (around which the apparatus is worn) and light reflected from the part of the user's appendage may pass through the covering element and be detected by the optical sensing elements. The apparatus 400 is provided in a bracelet type form factor and comprises an attachment mechanism 412 such as a clasp or fastening mechanism to enable apparatus to be releasable attached and wrapped around the user's appendage.

Figure 4B shows the apparatus 400 when in a second/opaque/non in use state wherein the covering element 404 is in a substantially opaque/non transparent so as to be in a blocking state. In this state, the various components underlying the covering element, not least the light sources and optical sensing elements, are not visible and are hidden/concealed from view, or are at least of reduced visibility to a viewer/user.

Figure 5 shows a further example of an apparatus 500 according to present disclosure in which a plurality of light sources 502R and 502G are provided and a plurality of optical sensing elements 503R and 503G are provided, all of which are covered by covering element 504. A controller 513 may be provided to control the adjustable transmissivity of the covering element 504, e.g. controlling the application of a voltage/electric field to the covering element. The controller can be configured so as to control the transmissivity of the covering element and affect switching of the transmissivity of the covering element between a first transmissive mode and a second opaque/blocking mode in response to receipt of a signal, such as a signal indicating whether or not the apparatus is in use, being worn, or if the light source and optical sensor are being activated/in a powered state or if a light sensing measurement is to be made.

The apparatus may further comprise one or more frequency dependent optical filters, such as colour filters 511R and 512G. The colour filters may be configured such that only light of a predetermined range of wavelengths may pass through the filter. In some examples, one or more filters may be provided that only permit the passage of particular colours, e.g. red light, green light, blue light or infra-red light.

The emission of light of a particular wavelength may be achieved either via a light source in combination with a filter of a particular wavelength to be outputted. Alternatively, a light source may be provided that only generates and emits light of the predetermined range of wavelength.

The provision of a colour filter for the optical sensing element means that only light of a predetermined range of wavelengths may be incident to and detected by the optical sensing element. This may reduce the detrimental effect during a PPG sensor measurement of detecting ambient light, i.e. over a whole range of wavelengths, which might otherwise oversaturate, interfere with and swamp the detection of just the emitted light which is reflected from a region of the user and the ability to detect the small variations of light intensity (0.5-5% variation) caused by blood pulses.

The filter may be integral with the covering element, i.e. be comprised with the covering element, for example such that the transmissive first state of the covering element is configured such that it only permits light of a predetermined wavelength therethrough, whereas in the second blocking/opaque state the covering element does not permit light of any wavelength therethrough. In such examples, whilst the covering element may have a coloured appearance corresponding to the filtered coloured wherein the first state, in the second opaque state, the covering element may have a black appearance, thereby concealing the colour from view as well as concealing from view any underlying components, such as the light source and optical sensing element.

The apparatus may be configured so as to provide:
at least a first frequency dependent optical filter 511 R that permits the passage of light only of a first predetermined range (e.g. red light), and
at least a second frequency dependent optical filter 512G that permits light of only a second predetermined range of wavelengths (e.g. green light) different to the first range.

Such a configuration enables the use of multi coloured filters in the apparatus which my assist PPG measurements, but which can be made non-visible to a user.

For example, the apparatus 500 may be configured so as to emit red light 508R. Such emitted red light may be reflected from a user's body and the reflected light 508R' may be detected by optical sensing element 503R. A further light source 502G may be configured so as to emit green light 508G and a further optical sensing element 503G may be configured so as to only detect green light 508G', i.e. light of other wavelengths such as red light 508'R is blocked from being detected by the optical sensing element by the colour filter 512G.

In other examples, the apparatus may be configured so as to selectively emit and detect light within two or more frequency ranges, at least for example: red, green, blue and infra-red parts of the electromagnetic spectrum. In some examples other wavelength ranges may be used.

The ability to make use of several different discrete wavelength ranges (i.e. several colours of light) in a PPG measurement may enable additional information, such as pulse oxygenation to be determined. Also, it can assist motion artefact cancellation. Furthermore, it can enable the frequency/colour of the 'probing light' that is used for PPG measurements to be optimised/adapted for differing users' skin tones.

Examples of the apparatus may enable the use of multi coloured filters but whilst also enabling the colour filters to be non-visible to a user when the apparatus is not in use / being worn. Such visible colour filters might otherwise adversely affect the aesthetics of the apparatus and affect the design freedom of the apparatus in accommodating the visible and differently coloured parts of the apparatus. For example a designer might be limited in the materials and colours he can use and select that complement the visible and differently coloured parts of the apparatus.

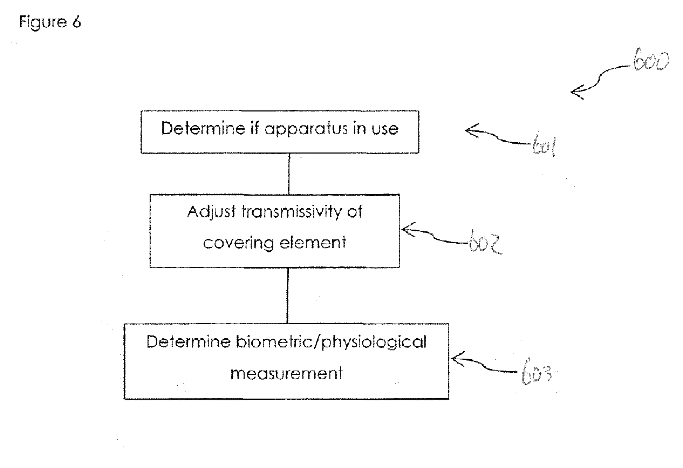

Figure 6 schematically illustrates a flow chart of a method 600 according to an example of the present disclosure.

In block set 602 the transmissivity of a covering element is adjusted. The adjustment of block 602 may be in response to a determination as to whether or not the apparatus is in use, as shown in block 601. Block 601 may involve determining whether or not a sensor measurement is starting to be taken and/or if the light source and optical sensing element are operational, activated and/or powered in order to obtain a sensor measurement. If it is determined that the apparatus is in use, the covering element may be switched to a transmissive state. Whereas, if it is determined that the apparatus is not in use, the covering element may be switched to a non-transmissive/optically blocking/opaque state.

If it is determined that the apparatus is in use and the transmissivity of the covering element has being switched to a transmissive state, the method may further include block 603 in which a biometric/physiological measure may be determined, not least for example via photoplethysmography.

In accordance with a further example of an apparatus in the present disclosure, an apparatus may be provided comprising means configured to cause/enable the apparatus at least to perform one or more of the above method blocks.

The component blocks of Figure 6 are functional and the functions described may or may not be performed by a single physical entity. For example, the apparatus might not itself determine and calculate a biometric/physiological measurement, but instead may send its light sensing measurements to a remote device (e.g. mobile phone, tablet, laptop/desktop computer or server) which processes the light sensor measurements and determines/calculates a biometric/physiological measurement therefrom.

The flowchart of Figure 6 represents one possible scenario among others. The order of the blocks shown is not absolutely required, so in principle, the various blocks can be performed out of order. Not all the blocks are essential. For example, blocks 601 and 603 may be optional. In certain examples one or more blocks may be performed in a different order or overlapping in time, in series or in parallel one or more blocks may be omitted or added or changed in some combination of ways.

Although examples of the apparatus have been described in terms of comprising various components, it should be understood that the components may be embodied as or otherwise controlled by a corresponding processing element or processor of the apparatus. In this regard, each of the components described may be one or more of any device or means that is configured to perform the corresponding functions of the respective components as described above.

In some examples, the apparatus may be embodied on a wearable portable electronic device, such as a wrist device, computing device that may additionally provide one or more audio/text/video communication functions (e.g. tele-communication, video-communication, and/or text transmission (Short Message Service (SMS)/ Multimedia Message Service (MMS)/emailing) functions), interactive/non-interactive viewing functions (e.g. web-browsing, navigation, TV/program viewing functions), music recording/playing functions (e.g. Moving Picture Experts Group-1 Audio Layer 3 (MP3) or other format and/or (frequency modulation/amplitude modulation) radio broadcast recording/playing), downloading/sending of data functions, image capture function (e.g. using a (e.g. in-built) digital camera), and gaming functions.

The apparatus may be provided in a module. As used here 'module' refers to a unit or apparatus that excludes certain parts/components that would be added by an end manufacturer or a user.

Features described in the preceding description may be used in combinations other than the combinations explicitly described.

Although functions have been described with reference to certain features, those functions may be performable by other features whether described or not. Although features have been described with reference to certain examples, those features may also be present in other examples whether described or not. Although various examples of the present disclosure have been described in the preceding paragraphs with reference to various examples, it should be appreciated that modifications to the examples given can be made without departing from the scope of the invention as set out in the claims.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one ..." or by using "consisting".

In this description, reference has been made to various examples. The description of features or functions in relation to an example indicates that those features or functions are present in that example. The use of the term 'example' or 'for example' or 'may' in the text denotes, whether explicitly stated or not, that such features or functions are present in at least the described example, whether described as an example or not, and that they can be, but are not necessarily, present in some or all other examples. Thus 'example', 'for example' or 'may' refers to a particular instance in a class of examples. A property of the instance can be a property of only that instance or a property of the class or a property of a sub-class of the class that includes some but not all of the instances in the class.

In this description, references to "a/an/the" [feature, element, component, means...] are to be interpreted as "at least one" [feature, element, component, means...] unless explicitly stated otherwise.

The above description describes some examples of the present disclosure however those of ordinary skill in the art will be aware of possible alternative structures and method features which offer equivalent functionality to the specific examples of such structures and features described herein above and which for the sake of brevity and clarity have been omitted from the above description. Nonetheless, the above description should be read as implicitly including reference to such alternative structures and method features which provide equivalent functionality unless such alternative structures or method features are explicitly excluded in the above description of the examples of the present disclosure.

Whilst endeavouring in the foregoing specification to draw attention to those features of examples of the present disclosure believed to be of particular importance it should be understood that the applicant claims protection in respect of any patentable feature or combination of features hereinbefore referred to and/or shown in the drawings whether or not particular emphasis has been placed thereon.

## Claims

1. An apparatus comprising:
a support structure configured to attach to an appendage of a user, wherein the support structure comprises:
a light source;
an optical sensing element; and
a covering element arranged to cover at least the optical sensing element, wherein the covering element is configured such that the covering element's transmissivity is adjustable.

2. The apparatus of any one or more of the previous claims, wherein the covering element is further arranged to cover the light source.

3. The apparatus of any one or more of the previous claims, wherein the covering element comprises a material configured so as to be switchable between at least a first transmissivity state and a second transmissivity state.

4. The apparatus of previous claim 3, wherein the covering element is configured such that one or more of:
the covering element is able to be in the first state when the apparatus is in use and the covering element is able to be the second state when the apparatus is not in use;
the first state is substantially transparent and the second states substantially opaque;
in the first state, light may pass through the covering element for detection by the optical sensing element; and
in the second state, the optical sensing element is not visible through the covering element.

5. The apparatus of any one or more of the previous claims, wherein the covering element comprises one of more of the following:
smart glass;
a photochromic material;
a thermochromic material;
an electrochromic material;
a liquid crystal;
a suspended particle device.

6. The apparatus of any one or more of the previous claims, further comprising a controller to control the transmissivity of the covering element.

7. The apparatus of any one or more of the previous claims, wherein the covering element is configured to at least one or more of:
form an external part of a housing of the apparatus;
form a window through a housing of the apparatus;
form a protective barrier to the optical sensing element and/or a light source of the apparatus;
seal, within a housing of the apparatus, the optical sensing element and/or a light source of the apparatus.

8. The apparatus of any one or more of the previous claims, further comprising a frequency dependent optical filter.

9. The apparatus of claim 8, wherein the apparatus further comprises at least a second frequency dependent optical filter.

10. The apparatus of any one or more of the previous claims, wherein the apparatus is configured such that light from the light source is directed to a part of the user's appendage.

11. The apparatus of claim 10, wherein the apparatus is configured such that, in use, light reflected from the user's appendage is detected by the optical sensing element.

12. The apparatus of any one or more of the previous claims, wherein the apparatus is configured for use in determining one or more of:
a biometric measurement, a physiological measurement, a photoplethysmogram measurement, a volumetric measurement, a blood oxygen saturation measurement, a cardiovascular measurement, a heart rate measurement or a pulse oxygenation measurement.

13. A module or device comprising the apparatus of any one or more of previous claims.

14. A method comprising causing, at least in part, actions that result in:
adjusting a transmissivity of a covering element of an apparatus, wherein the apparatus comprises:
a support structure configured to attach to an appendage of a user, wherein the support structure comprises:
a light source;
an optical sensing element; and
wherein the covering element is arranged to cover at least the optical sensing element.

15. The method of claim 14 comprising causing, at least in part, actions that result in one or more of:
determining if apparatus is in use; and
determining biometric/physiological measurement.
